# EUROPÄISCHE PATENTSCHRIFT

(11) **EP 3 310 426 B1**
(45) Veröffentlichungstag und Bekanntmachung des Hinweises auf die Patenterteilung: **23.03.2022**
(21) Anmeldenummer: 16730376.7
(22) Anmeldetag: 17.06.2016
(51) Int. Cl.: A61M 39/10

(54) **MÄNNLICHER UND WEIBLICHER LUER-VERBINDER UND LUER-VERBINDUNGSSYSTEM**
MALE AND FEMALE LUER CONNECTOR AND LUER CONNECTION SYSTEM
CONNECTEUR MALE ET FEMELLE DE TYPE LUER ET SYSTEME DE CONNEXION DE TYPE LUER

(30) Priorität: 19.06.2015 DE 102015211370
(43) Veröffentlichungstag der Anmeldung: 25.04.2018
(73) Patentinhaber: Schedler, Michael, 66877 Ramstein-Miesenbach (DE); Müller, Christopher, 67659 Kaiserslautern (DE)
(72) Erfinder: Schedler, Michael, 66877 Ramstein-Miesenbach (DE); Müller, Christopher, 67659 Kaiserslautern (DE)
(74) Vertreter: Mader, Joachim
(86) Internationale Anmeldenummer: PCT/EP2016/064049
(87) Internationale Veröffentlichungsnummer: WO 2016/202999

(56) Entgegenhaltungen:
- WO-A1-2008/009946
- WO-A1-2014/049775
- DE-A1-102011 011 762
- US-A1- 2009 099 552

## Beschreibung

### 1. Technisches Gebiet

Die vorliegende Erfindung betrifft männliche und weibliche Luer- und Luer-Lock-Verbinder und ein Verbindungssystem aus je einem männlichen und einem weiblichen Luer- oder Luer-Lock-Verbinder, die Verwechslungen zwischen Luer-Verbindern ausschließen.

### 2. Technischer Hintergrund

Luer-Verbindungssysteme, die männliche und weibliche Luer-Verbinder aufweisen, sind im Stand der Technik in unterschiedlichen Ausführungen bekannt. Luer-Verbinder sind international in der Norm ISO 594 bzw. europaweit in der EN 20594 (bzw. EN 1707) und deutschlandweit in der DIN EN 20594 normiert. Das Luer-Verbindungssystem ermöglicht eine Herstellung von herstellerunabhängigen sicheren, dichten und sterilen Verbindungen insbesondere im medizintechnischen Bereich. Die genormten Standard-Luer-Verbinder unterschiedlicher Hersteller sind beliebig miteinander kombinierbar. Beispielsweise können mit dem Luer-Verbindungssystem Spritzen und Kanülen unterschiedlicher Hersteller beliebig miteinander kombiniert werden.

Ein männlicher Standard-Luer-Verbinder weist einen Rohrkörper auf, der einen konischen Außenmantel aufweist. Dieser konische Außenmantel hat nach der Norm von der Spitze zur Basis eine 6%-Steigung. Der weibliche Standard-Luer-Verbinder weist einen hohlen Aufnahmekörper auf, der eine konische hülsenförmige Aufnahme aufweist, die ebenfalls eine 6%-Steigung aufweist. Am äußeren Ende des weiblichen Standard-Luer-Verbinders weist dieser einen Bund auf, der oft radial nach außen absteht. Der weibliche Standard-Luer-Verbinder lässt sich auf den männlichen Standard-Luer leicht aufsetzen und per Reibschluss sicher und gasdicht verbinden. Ebenso leicht kann die Verbindung wieder gelöst werden.

Neben dem gewöhnlichen Standard-Luer-Verbindungssystem sind auch sogenannte Luer-Lock-Verbindungssysteme im Stand der Technik bekannt. Diese weisen am männlichen Luer-Verbinder zusätzlich ein Gewinde auf in das radial vorstehende Verriegelungselemente eingreifen können, um den männlichen und den weiblichen Luer-Verbinder mittels einer Drehung verriegeln. Damit ist die Luer-Verbindung zugfest ausgestaltet.

Üblicherweise befinden sich weibliche Luer-Verbinder an einer Kanüle oder einem Katheter, oder am Eingang einer Leitung. Männliche Luer-Verbinder befinden sich meist an einer Spritze, einem Dosierbehälter, einem medizinischen Gerät oder am Ausgang einer Leitung.

Die im Stand der Technik bekannten Luer-Verbindungssysteme haben den Nachteil, dass es aufgrund der gewünschten Kompatibilität der einzelnen Verbinder untereinander erhebliche Verwechslungsrisiken gibt. So sollte ursprünglich das Luer-Verbindungssystem nach den oben genannten Normen zunächst nur für die Verbindung zwischen Einmalspritzen bzw. Injektionskanülen und anderen medizinischen Geräten, z. B. Transfusionsgeräten, verwendet werden. Seine einfache, bequeme und sichere Handhabung führten jedoch zu einer breitgefächerten Verwendung auch für andere medizinische Anwendungen. Verwechslungen, die sich aus falschen Verbindungen ergeben, können für den Patienten lebensgefährlich sein. Da das Luer-Verbindungssystem für unterschiedlichste Anwendungen wie vaskuläre und spinale bzw. peridurale, teilweise auch für enterale (z.B. Ernährungssonden) und respiratorische Anwendungen, gleich gestaltet ist, besteht die Gefahr, dass ein venös zu injizierendes Medikament fälschlicherweise spinal appliziert wird. Beispielsweise besteht die Gefahr, dass ein intravenösen Zugang, an dem sich ein weiblicher Luer-Verbinder befindet, fälschlicherweise mit einer Leitung mit männlichem Luer-Verbinder verbunden wird, an dem ein enteral zu verabreichendes Medikament ansteht.

Derartige Verwechslungen und anderen Probleme wurde unter anderem in dem Fachbericht der DIN (DIN-Fachbericht 88:2000) aus dem Jahr 2000 mit dem Titel "LUER-Verbindungen - Ein Bericht der CEN Forum Task Group "LUER fittings"; Deutsche Übersetzung des CR 13825:2000" diskutiert.

Aus der WO 2008/009946 A1 ist Luer-Verbinder-System bekannt, bei dem ein männlicher Luer-Verbinder sich nur mit einem besonders codierten weiblichen Luer-Verbinder verbinden kann.

Aus der EP 1 083 963 A1 ist ein spezielles Verbindungssystem mit Sicherheitsindex für Flüssigkeiten im medizinischen Umfeld bekannt.

Die zu lösende technische Aufgabe der vorliegenden Erfindung ist es daher, das Luer-Verbindungssystem und ihre männlichen und weiblichen Luer-Verbinder so zu verbessern, dass eine Verwechslung verschiedener medizinischer Systeme in der Anwendung möglichst kostengünstig und ohne aufwändige Veränderungen der bestehenden Verbindungssysteme ausgeschlossen wird.

### 3. Inhalt der Erfindung

Die oben genannte Aufgabe wird erfindungsgemäß gelöst durch einen männlichen Luer-Verbinder gemäß Anspruch 1, einen weiblichen Luer-Verbinder gemäß Anspruch 9 sowie durch ein Verbindungssystem aus einem männlichen und einem weiblichen Luer-Verbinder nach Anspruch 12.

Insbesondere wird die oben genannte Aufgabe durch einen männlichen Luer-Verbinder gelöst, aufweisend einen Rohrkörper mit einem konischen Außenmantel und einem Verbindungssperrelement wobei das Verbindungssperrelement einen Raum um den Rohrkörper herum begrenzt und in axialer Richtung einen Codierungsanschlag bildet. Das Verbindungssperrelement des männlichen Luer-Verbinders ist durch seinen in axialer Richtung angeordneten Codierungsanschlag dazu eingerichtet, eine Verbindung des männlichen Luer-Verbinders mit einem weiblichen Standard-Luer-Verbinder oder weiblichen Standard-Luer-Lock-Verbinder mechanisch zu verhindern, aber eine Verbindung mit einem passenden weiblichen Luer-Verbinder, welcher invers geformt ist, zu ermöglichen. Der Codierungsanschlag des männlichen Luer-Verbinders ist komplementär zu einem Codierungseinschnitt des passenden weiblichen Luer-Verbinders ausgestaltet, so dass diese Codierungselemente nach dem "Schlüssel-Schloss-Prinzip" ein Zusammenfügen erfindungsgemäßer Luer-Verbinder erlauben oder verhindern. Beim Zusammenfügen passender Luer-Verbinder kann sich der Codierungsanschlag an dem Codierungseinschnitt vorbei bewegen. Das Schlüssel-Schloss-Prinzip der erfindungsgemäßen Luer-Verbinder hat den Vorteil, dass Verwechslungen von Luer-Verbindern, die beispielsweise gleichzeitig bei einem Patienten verwendet werden, ausgeschlossen sind. Insbesondere verhindert ein erfindungsgemäßer männlicher Luer-Verbinder für beispielsweise ein enteral zu verabreichendes Medikament, dass dieser fälschlicherweise mit einem intravenösen Zugang mit einem weiblichen Standard-Luer-Verbinder verbunden wird. Der Codierungsanschlag verhindert hierbei ein Aufstecken eines weiblichen Standard-Luer-Verbinders.

Vorzugsweise haben Luer-Verbindungssysteme, die für ein bestimmtes Einsatzgebiet verwendet werden, eine spezielle Codierung. Ein entsprechender Codierungsanschlag weist daher eine definierte geometrische Form auf. Damit wird sichergestellt, dass Verwechslungen der Luer-Verbindungssysteme miteinander unmöglich sind, da nur diejenigen weiblichen Luer-Verbinder mit dem codierten männlichen Luer-Verbinder verbunden werden können, die eine entsprechende Form haben, die zu dem Codierungsanschlag des männlichen Luer-Verbinders passt.

Das Verbindungssperrelement des männlichen Luer-Verbinders kann bevorzugt zusammen mit dem Rohrkörper spritzgegossen werden, so dass bestehende Produktionsverfahren für diese Verbinder beibehalten werden können.

Da das Verbindungssperrelement lediglich einen Raum um den Rohrkörper herum begrenzt und einen Codierungsanschlag bildet, kann der Rohrkörper mit seinem konischen Außenmantel unverändert nach dem Luer-Standard ausgeführt werden. Es ergeben sich daher keinerlei Probleme im Hinblick auf die Dichtheit der Verbindung oder ähnliches.

Bevorzugt besteht das Verbindungssperrelement aus mindestens einem mit dem Rohrkörper starr verbundenen Vorsprung besteht, der sich axial in einen rohrförmigen Raum um den Rohrkörper herum erstreckt. Die Verhinderung der Verbindung über einen oder mehrere Vorsprünge ist besonders vorteilhaft, da hier auf einfache Art und Weise mechanisch eine Kopplung von unpassenden männlichen und weiblichen Luer-Verbindern verhindert wird. Der weibliche Standard-Luer-Verbinder stößt beim Versuch der Verbindung an den Vorsprung des männlichen Luer-Verbinders an und kann diesen mechanisch nicht überwinden. Weiterhin lassen sich solche Vorsprünge, die sich axial in einen rohrförmigen Raum um den Rohrkörper herum erstrecken, leicht fertigen, beispielsweise spritzgießen, und benötigen nur wenig zusätzliches Material.

Bevorzugt ist der männliche Luer-Verbinder ein männlicher Luer-Lock-Verbinder und weist weiterhin einen Gewindekörper mit einem Innengewinde aufweist, wobei der Codierungsanschlag des Verbindungssperrelements am äußeren Ende des Gewindekörpers angebracht ist. Auch bei einem männlichen Luer-Lock-Verbinder mit Innengewinde verhindert das Verbindungssperrelement mit seinen Codierungsflächen die mechanische Verbindung mit einem unpassenden weiblichen Luer-Lock-Verbinder oder einem Standard-Luer-Lock-Verbinder.

Bevorzugt ist das Verbindungssperrelement ein separat hergestelltes, bevorzugt kappenförmiges Bauteil, das mit dem männlichen Luer-Verbinder verbunden ist. Damit kann das Verbindungssperrelement einfach an bestehende Einrichtungen oder Geräte mit Luer-Anschluss nachgerüstet werden. Hierzu wird das separat hergestellte Verbindungssperrelement an dem bestehenden männlichen Luer-Verbinder befestigt beispielsweise durch Aufstecken, durch Ankleben, Verschrauben, Verschweißen oder durch ähnliche Befestigungsmaßnahmen. Wenn das Verbindungssperrelement bevorzugt ein kappenförmiges Bauteil ist kann es beispielsweise einfach auf eine Hülse um den eigentlichen männlichen Luer-Konus herum aufgesteckt werden. Zur Sicherung kann diese Verbindung zusätzlich verklebt oder verschweißt sein. Die Hülse kann gleichzeitig der Gewindekörper eines männlichen Luer-Lock-Verbinders sein.

Bevorzugt weist das Verbindungssperrelement zwei gegenüberliegende Codierungsanschläge auf. Damit kann besonders vorteilhaft eine mechanische Verbindung codiert werden, die auch durch den Versuch eines schrägen Verbindens nicht fälschlicherweise verbunden werden kann. Damit wird die Verwechslungssicherheit weiterhin erhöht.

Bevorzugt werden die zwei gegenüberliegenden Codierungsanschläge durch zwei gegenüberliegende Vorsprünge geformt.

Bevorzugt sind die Codierungsanschläge in ihrer Form gleich ausgestaltet. Damit kann die Luer-Verbindung in zwei verschiedenen axialen Winkelstellungen der Verbinder zueinander verbunden werden.

Alternativ sind die Codierungsanschläge in ihrer Form unterschiedlich ausgestaltet. Damit sind mehr Codierungsmöglichkeiten vorhanden, um mehr unterschiedliche Verbindungssysteme für unterschiedliche Anwendungen zu codieren.

Bevorzugt weist der Codierungsanschlag, in axialer Richtung gesehen, eine gerade, dreieckige, rechteckige, kreisabschnittsförmige oder trapezförmige Silhouette auf. Diese Formen des Codierungsanschlags ergeben auch bei den vorliegenden kleinen Abmessungen von Luer-Verbindern eine sichere Codierung.

Bevorzugt ist das Verbindungssperrelement separat vom Hohlkörper hergestellt und weist eine hohlzylindrische Aufnahme auf, mit der das Verbindungssperrelement auf dem Gewindekörper des männlichen Luer-Lock-Verbinders verbindbar ist. Dies hat den Vorteil, dass das Verbindungssperrelement einfacher separat hergestellt werden kann als zusammen mit dem Gewindekörper des Luer-Lock-Verbinders. Dies führt zu Einsparungen in der Produktion solcher männlicher Luer-Lock-Verbinder.

Alternativ ist das Verbindungssperrelement bevorzugt einstückig mit dem männlichen Luer-Verbinder ausgebildet. Dies ist insbesondere vorteilhaft, da eine einstückige Ausbildung des erfindungsgemäßen männlichen Luer-Verbinders besonders robust ist und ein Lösen des Verbindungssperrelements von dem männlichen Luer-Verbinder unmöglich ist. Daneben entfallen bei einer einstückigen Ausbildung jegliche Montagevorgänge.

Die oben genannte Aufgabe wird auch gelöst durch einen weiblichen Luer-Verbinder, aufweisend einen hohlen Aufnahmekörper mit einer konischen Aufnahme, und einen Bund am äußeren Ende des Aufnahmekörpers, wobei der Bund mindestens einen Codierungseinschnitt aufweist.

Die Codierungseinschnitte des weiblichen Luer-Verbinders sind so eingerichtet, dass der weibliche Luer-Verbinder mit einem oben beschriebenen hierzu passenden männlichen Luer-Verbinder verbunden werden kann. Besonders vorteilhaft ist es dabei, dass der weibliche Luer-Verbinder auch mit einem männlichen Standard-Luer-Verbinder verbunden werden kann. Dennoch ist es durch die Codierung des erfindungsgemäßen weiblichen Luer-Verbinders über den Codierungseinschnitt möglich, gefährliche Verwechslungen der Luer-Verbindung zu verhindern.

Bevorzugt ist der weibliche Luer-Verbinder ein weiblicher Luer-Lock-Verbinder und weist weiterhin ein oder mehrere radial vorstehende Verriegelungselemente auf, die an dem Bund des weiblichen Luer-Verbinders angeordnet sind. Das eine oder die mehreren Verriegelungselemente sind so eingerichtet, dass sie mit einem Gewinde eines männlichen Luer-Lock-Verbinders einen Formschluss bilden können und insbesondere mit dem Gewinde des männlichen Luer-Lock-Verbinders drehbar verriegeln können.

Bevorzugt weist der Codierungseinschnitt in axialer Richtung gesehen eine gerade, dreieckige, rechteckige, kreisabschnittsförmig oder trapezförmige Silhouette auf. Die Codierungseinschnitte des weiblichen Luer-Verbinders sind bevorzugt invers zu den Codierungsanschlägen des oben beschriebenen männlichen Luer-Verbinders ausgestaltet. Die Codierungseinschnitte sind damit so ausgestaltet, dass sie eine zu den Codierungsabschnitten des männlichen Luer-Verbinders inverse bzw. passende Silhouette aufweisen.

Bevorzugt weist der Bund des weiblichen Luer-Verbinders zwei radial gegenüberliegend angeordnete Codierungseinschnitte auf. Damit ist eine sichere Codierung sichergestellt, so dass auch bei einem schrägen Aufsetzen des weiblichen Luer-Verbinders ein unpassender weiblicher Luer-Verbinder nicht mit dem männlichen Luer-Verbinder verbunden werden kann.

In einer weiteren bevorzugten Ausführungsform weist der Bund des weiblichen Luer-Verbinders zwei asymmetrisch angeordnete Codierungseinschnitte auf. Für spezielle Anwendungen können die Codierungseinschnitte auch asymmetrisch bzw. nicht spiegelbildlich, beispielsweise im 90°-Winkel zueinander, am Bund des weiblichen Luer Verbinders angeordnet sein. Damit ist eine Verdrehsicherheit beispielsweise für die Verbindung bei OP-Instrumenten gegeben. Eine definierte axiale Winkelstellung beim Zusammenfügen von weiblichem und männlichem Luer-Verbinder wird auch bei der bevorzugten Verwendung von nur einem Codierungseinschnitt oder bei mehreren unterschiedlich geformten Codierungseinschnitten erhalten.

Die oben genannte Aufgabe wird auch gelöst durch ein Verbindungssystem aufweisend einen männlichen Luer-Verbinder wie oben beschrieben und einen weiblichen Luer-Verbinder wie oben beschrieben, wobei der eine Codierungseinschnitt oder die mehreren Codierungseinschnitte des weiblichen Luer-Verbinders invers zum einen Codierungsanschlag oder den mehreren Codierungsanschlägen des männlichen Luer-Verbinders ausgestaltet ist oder sind.

### 4. Beschreibung der Figuren

Im Folgenden wird die vorliegende Erfindung anhand der beiliegenden Figuren näher beschrieben. Es zeigt:
- Fig. 1A bis 1D: ein Ausführungsbeispiel eines erfindungsgemäßen weiblichen LuerVerbinders in vier verschiedenen Perspektiven;
- Fig. 2A bis 2C: ein Ausführungsbeispiel eines erfindungsgemäßen LuerVerbindungssystems aus einem männlichen und einem weiblichen Luer-Verbinder in mehreren Perspektiven im verbundenen Zustand;
- Fig. 3A und 3B: ein Ausführungsbeispiel eines erfindungsgemäßen Luer-LockVerbindungssystems aus einem männlichen und einem weiblichen Luer-Lock-Verbinder;
- Fig. 4A bis 4D: das Ausführungsbeispiel des erfindungsgemäßen Luer-LockVerbindungssystems von Fig. 3 in dem verbundenen Zustand in mehreren Perspektiven;
- Fig. 5: ein weiteres Ausführungsbeispiel eines erfindungsgemäßen LuerVerbindungssystems aus einem männlichen und einem weiblichen Luer-Lock-Verbinder;
- Fig. 6A bis 6C: das Ausführungsbeispiel des erfindungsgemäßen LuerVerbindungssystems von Fig. 5 in dem verbundenen Zustand in mehreren Perspektiven;
- Fig. 7: ein weiteres Ausführungsbeispiel eines erfindungsgemäßen LuerVerbindungssystems aus einem männlichen und einem weiblichen Luer-Verbinder;
- Fig. 8A bis 8C: das Ausführungsbeispiel des erfindungsgemäßen LuerVerbindungssystems von Fig. 7 in dem verbundenen Zustand in mehreren Perspektiven;
- Fig. 9: ein weiteres Ausführungsbeispiel eines erfindungsgemäßen Luer-LockVerbindungssystems aus einem männlichen und einem weiblichen Luer-Lock-Verbinder;
- Fig. 10A bis 10D: das Ausführungsbeispiel des erfindungsgemäßen Luer-LockVerbindungssystems von Fig. 9 in dem verbundenen Zustand in mehreren Perspektiven;
- Fig. 11A bis 11D: axiale Draufsichten auf Ausführungsbeispiele des erfindungsgemäßen Luer-Lock-Verbindungssystems mit unterschiedlichen Silhouetten der Codierungsanschläge und Codierungseinschnitte;
- Fig. 12: ein weiteres Ausführungsbeispiel eines erfindungsgemäßen LuerVerbindungssystems aus einem männlichen und einem weiblichen Luer-Verbinder; und
- Fig. 13A bis 13D: das Ausführungsbeispiel des erfindungsgemäßen LuerVerbindungssystems von Fig. 12 in dem verbundenen Zustand in mehreren Perspektiven.

### 5. Beschreibung bevorzugter Ausführungsbeispiele

Im Folgenden werden anhand der Figuren bevorzugte Ausführungsbeispiele der vorliegenden Erfindung erläutert:

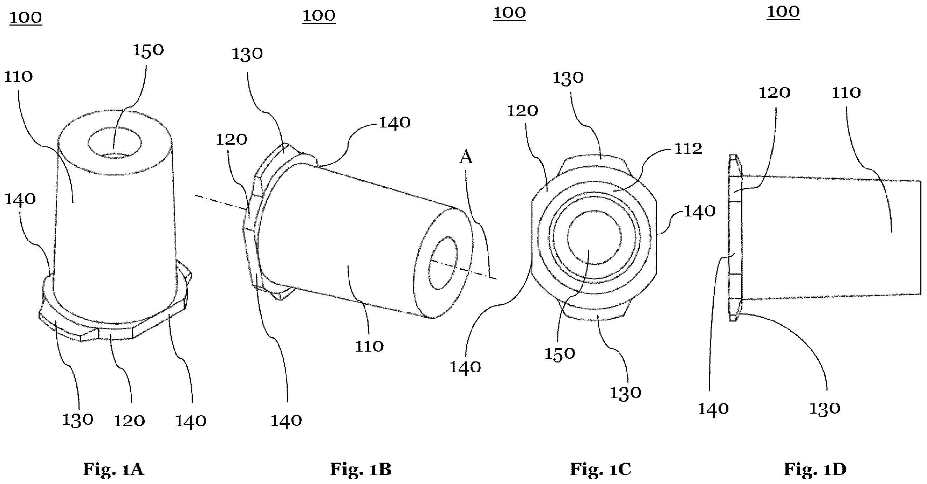

Figuren 1A bis 1D zeigen ein Ausführungsbeispiel eines weiblichen Luer-Verbinders 100 in vier verschiedenen Perspektiven. Der weibliche Luer-Verbinder 100 weist einen hohlen Aufnahmekörper 110 auf, der innen eine konische Aufnahme 112 umfasst. Die konische Aufnahme 112 weist die für Luer-Verbinder typische Steigung von 6% auf und dient der Aufnahme und Abdichtung mit einem entsprechenden männlichen Luer-Verbinder. Der weibliche Luer-Verbinder 100 weist weiterhin einen Bund 120 auf, der am offenen Ende des konischen Aufnahmekörper 110 angeordnet ist. Der Bund 120 ist bevorzugt im Wesentlichen kreisringförmig mit einem rechteckigen Querschnitt. Damit ist der weibliche Luer-Verbinder 100 grundsätzlich auch zu männlichen Standard-Luer-Verbindern (nicht dargestellt) kompatibel und kann auch mit solchen verbunden werden.

Weiterhin weist der Bund 120 zwei gegenüberliegende weibliche Codierungseinschnitte 140 auf, die in der Ausführungsform der Figuren 1A bis 1D axial, also in Verbindungsrichtung gesehen, eine gerade Kante und somit eine gerade Silhouette aufweisen, wie in der axialen Ansicht von unten der Fig. 1C zu sehen. Die Codierungseinschnitte 140 werden daher durch zwei axial gegenüberliegende gerade Abschnitte des Bundes 120 gebildet.

Weiterhin weist der konische Aufnahmekörper 110 zwei radial vorstehende Verriegelungselemente 130 auf. Diese sind bevorzugt axial gegenüberliegend am Bund 120 des weiblichen Luer-Verbinders 100 angeordnet und nach außen konisch zulaufend oder abgeschrägt. Die Verriegelungselemente 130 dienen dazu, in ein Gewinde 322 eines männlichen Luer-Lock-Verbinders 300 einzugreifen und mit diesem durch eine Drehung gegen ein unbeabsichtigtes Lösen zu verriegeln.

Bei einem Luer-Verbindungssystem 400 mit einem männlichen Luer-Verbinder 200 ohne Gewinde 322, der auch als männlicher "Luer-Slip-Verbinder" bezeichnet wird, können die Verriegelungselemente 130 entfallen.

Der weibliche Luer-Verbinder 100 weist weiterhin eine Öffnung 150 am hinteren Ende des konischen Aufnahmekörpers 110 auf, an der beispielsweise Nadeln oder Leitungen (nicht dargestellt) angeschlossen sein können.

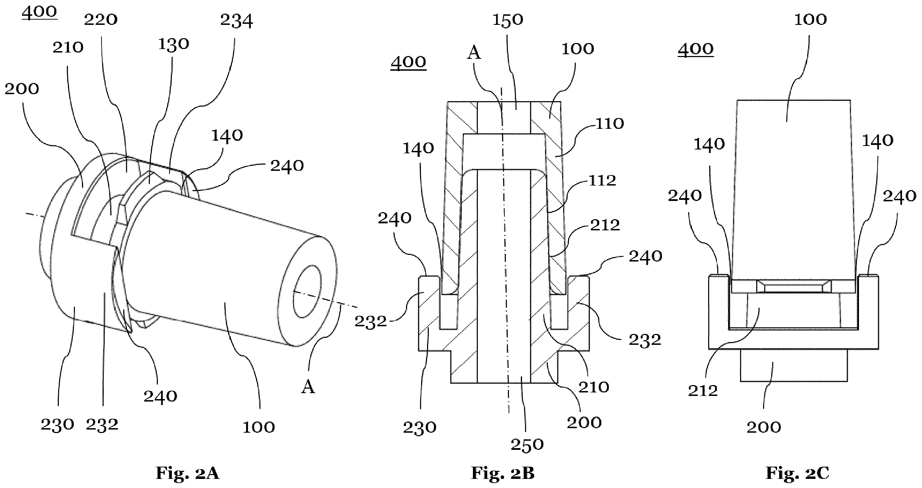

Die Figuren 2A bis 2C zeigen ein Ausführungsbeispiel eines Luer-Verbindungssystems 400 umfassend einen männlichen Luer-Verbinder 200 (hier ein Luer-Slip-Verbinder 200) und einen dazu passend codierten weiblichen Luer-Verbinder 100 im verbundenen Zustand. Der männliche Luer-Verbinder 200 weist einen Rohrkörper 210 auf. Dieser Rohrkörper hat einen konischen Außenmantel 212, der eine für Luer-Verbinder typische Steigung von 6% aufweist und zur Verbindung und Abdichtung mit einem entsprechenden weiblichen Luer-Verbinder 100 dient. Weiterhin weist der Rohrkörper 210 eine Durchgangsöffnung 250, auf, die sich, wie in Fig. 2B ersichtlich, durch den gesamten Rohrkörper 210 hindurch erstreckt und eine Leitung für den Durchfluss von Flüssigkeiten oder Gasen bereitstellt.

Weiterhin weist der männliche Luer-Verbinder 200 ein Verbindungssperrelement 230 auf, das in der Ausführungsform der Figuren 2A bis 2C als zwei gegenüberliegende Vorsprünge 232 ausgebildet ist. Diese Vorsprünge 232 sind mit dem Rohrkörper 210 starr verbunden und erstrecken sich axial in einen Raum um den konischen Rohrkörper 210 herum und begrenzen diesen Raum. Insbesondere bilden die zwei Vorsprünge 232 in axialer Richtung zwei Codierungsanschläge 240. Diese Codierungsanschläge 240 sind in Richtung zum Rohrkörper 210 hin durch ihre Silhouette geometrisch codiert und erlauben damit, dass ein entsprechend geometrisch codierter weiblicher Luer-Verbinder 100 axial aufgeschoben kann. Uncodierte oder anders codierte weiblichen Luer-Verbinder stehen mit ihrem Bund 120 an den Codierungsanschlägen 240 an und können dann nicht mit dem männlichen Luer-Verbinder 200 verbunden werden. Durch diese mechanische Codierung von männlichen und weiblichen Luer-Verbindern 100, 200, 300 können Verwechslungen beim Anschluss medizinischer Geräte und Zugänge vermieden werden.

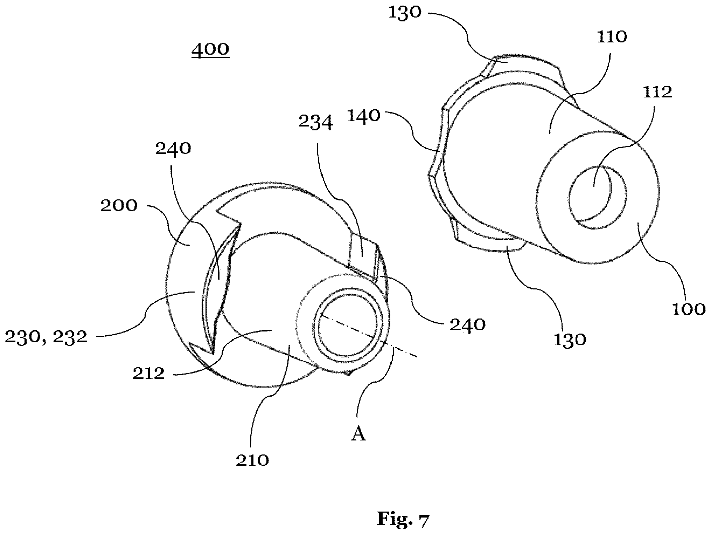

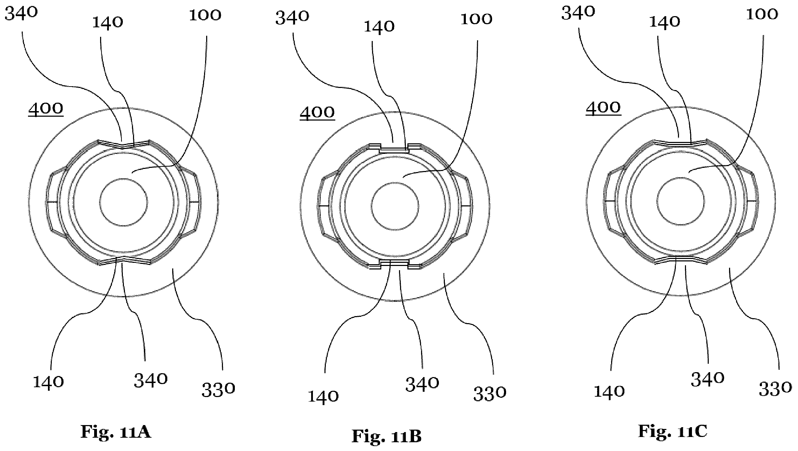

Bei der Ausführungsform der Figuren 2A bis 2C weisen die Codierungsanschläge 240, in Richtung zum Rohrkörper 210 hin, eine gerade Kante, also eine gerade Silhouette zur Codierung auf. Entsprechend weisen hierzu passende weibliche Luer-Verbinder 100 ebenfalls eine gerade Codierung auf, die durch eine gerade Silhouette des Codierungseinschnitts 140 gebildet wird. Ein entsprechender passender weiblicher Luer-Verbinder ist in den Figuren 1A bis 1D dargestellt. In den Figuren 7-9 sind Luer-Verbinder mit einer kreisabschnittsförmigen Silhouette des Codierungsanschlags 240, 340 und des Codierungseinschnittes 140 dargestellt. Die Figur 11A zeigt einen Luer-Verbinder mit einer dreieckigen Silhouette des Codierungsanschlags 340 und des Codierungseinschnittes 140. In der Figur 11B ist die Silhouette des Codierungsanschlags 340 und des Codierungseinschnittes 140 rechteckig. In der Figur 11C ist die Silhouette des trapezförmig. Die dargestellten zueinander passenden Paare aus Codierungsanschlag 240, 340 und Codierungseinschnitt 140 sind an gegenüberliegenden Seiten des Luer-Verbinders in ihrer Form gleich ausgebildet. Jedoch ist es auch möglich, dass die zueinander passenden Paare aus Codierungsanschlag 240, 340 und Codierungseinschnitt 140 an gegenüberliegenden Seiten des Luer-Verbinders in ihrer Form unterschiedlich ausgebildet sind, um die Codierungsmöglichkeiten zu erhöhen. Weiterhin ist es möglich nur jeweils ein zueinander passendes Paar aus Codierungsanschlag 240, 340 und Codierungseinschnitt 140 oder mehr als zwei Paare, etwa drei, vier oder fünf Paare, von zueinander passenden Paaren aus Codierungsanschlag 240, 340 und Codierungseinschnitt 140 an einem Luer-Verbindungssystem 400 vorzusehen.

Der männliche Luer-Verbinder 200 kann mit dem passend codierten weiblichen Luer-Verbinder 100 verbunden werden, da die weiblichen Codierungseinschnitte 140 und die männlichen Codierungsanschläge 240 invers zueinander ausgebildet sind. Beim Verbinden werden die Codierungseinschnitte 140 des passenden weiblichen Luer-Verbinders 100 an der Innenfläche 234 des Verbindungssperrelements 230 vorbeigeführt, wie in den Figuren 2B und 2C zu erkennen, so dass das Verbindungssperrelement 230 die Verbindung erlaubt. Der Codierungsanschlag 240 ist bevorzugt eine zur axialen Richtung senkrecht Fläche ausgebildet. Die Innenfläche 234 des Verbindungssperrelements 230 verläuft vorzugsweise parallel zur axialen Richtung. Die Innenfläche 234 kann aber auch in einem Winkel zur axialen Richtung angeordnet sein, beispielsweise mit einem Hinterschnitt, so dass der Raum sich hinter dem Codierungsanschlag 240 weitet.

Eine Verbindung des männlichen Luer-Verbinders 200 mit einem weiblichen Standard-Luer-Verbinder ist dementsprechend nicht möglich, da das Verbindungssperrelement 230 diese Verbindung mechanisch verhindert. In einem solchen Fall wird der weibliche Standard-Luer-Verbinder beim Versuch einer Verbindung an den Codierungsanschlägen 240 des Verbindungssperrelements 230 des männlichen Luer-Verbinders 200 anstehen und kann nicht weiter aufeinander zu bewegt werden.

In der Schnittansicht der Fig. 2 ist ersichtlich, dass der konische Außenmantel 212 des Rohrkörpers 210 mit der gleichermaßen konischen Aufnahme 112 des Aufnahmekörpers 110 gas- und flüssigkeitsdicht abdichtet. Dabei bilden die Durchgangsöffnung 250 des männlichen Luer-Verbinders 200 und die Öffnung 150 des weiblichen Luer-Verbinders 100 einen Kanal zum Flüssigkeitsaustausch von dem unteren zu dem oberen Ende der Luer-Verbindung 400.

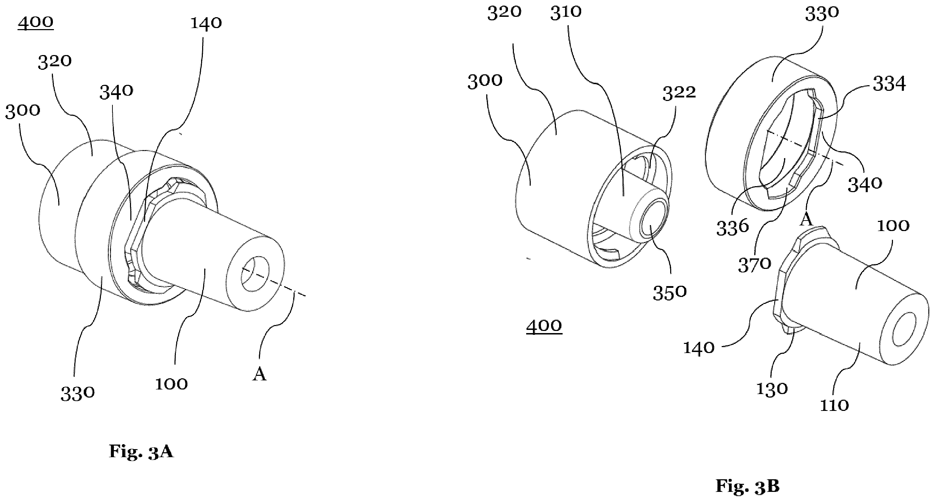

Die Figuren 3A - 4D zeigen ein weiteres Ausführungsbeispiel in Form eines Luer-Lock-Verbindungssystems 400, das aus einem männlichen 300 und einem weiblichen Luer-Lock-Verbinder 100 besteht. Die Fig. 3A zeigt das Verbindungssystem 400 im Zustand des Verbindens des weiblichen Luer-Verbinders 100 mit den männlichen Luer-Lock-Verbinder 300.

Fig. 3B zeigt das Ausführungsbeispiel von Fig. 3A als Explosionsansicht mit separaten Komponenten. In dieser Ausführungsform ist der Rohrkörper 310 einstückig mit einem Gewindekörper 320 ausgebildet, der hülsenförmig geformt ist und innenseitig mit einem Innengewinde 322 versehen ist. Das Verbindungssperrelement 330 ist ein separates kappenförmiges Bauteil, das eine hohlzylindrische Aufnahmeöffnung 336 aufweist, mit der es zur Befestigung auf dem Gewindekörper 320 des Luer-Lock-Verbinders 300 angeordnet werden kann. Das Verbindungssperrelement 330 umfasst bevorzugt zwei gegenüberliegende Codierungsanschläge 340, die sich nach der Montage am äußeren Ende des Gewindekörpers 320 befinden. Die Codierungsanschläge 340 codieren über die Form ihrer axial sichtbaren Silhouette die zur Verbindung erlaubten weiblichen Luer-Lock-Verbinder 100. Im Ausführungsbeispiel der Figuren 3A bis 4D ist die Silhouette gerade, so dass der weibliche Luer-Lock-Verbinder der Figuren 1A bis 1D mit ebenfalls gerader Silhouette passend ist.

Die Befestigung des Verbindungssperrelements 330 auf dem Gewindekörper 320 kann beispielsweise durch Kleben, Schweißen oder per Reibschluss erfolgen. Dann umfasst der männliche Luer-Lock-Verbinder 300 den Rohrkörper 310 mit dem Gewindekörper 320 und das Verbindungssperrelement 330 mit einem oder mehreren Codierungsanschlägen 340.

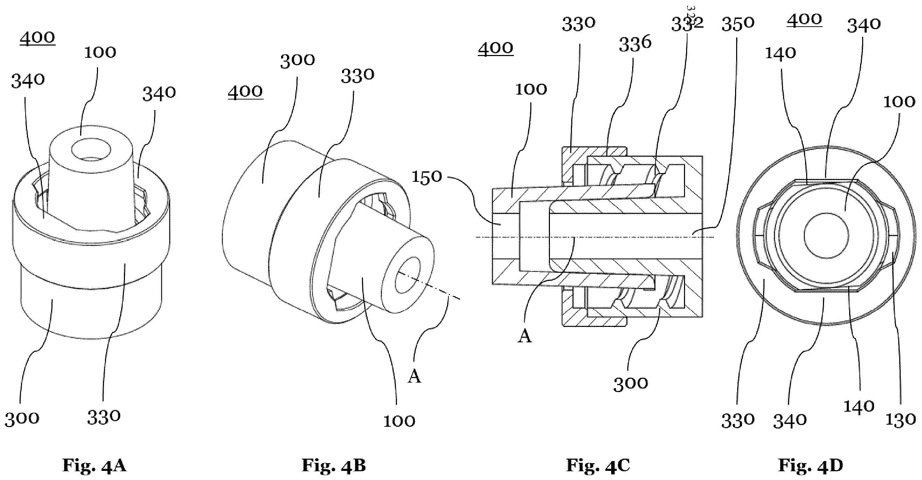

Zur Herstellung der Verbindung wird der weibliche Luer-Verbinder 100 durch das Verbindungssperrelement 330 des männlichen Luer-Lock-Verbinders 300 geführt und zwar insbesondere mit seinen Codierungseinschnitten 140 an den Innenflächen 334 der Codierungsanschläge 340 des Verbindungssperrelements 330 vorbeigeführt, wie in Fig. 3A, ersichtlich. Danach wird der weibliche Luer-Lock-Verbinder durch Einschrauben mit dem männlichen Luer-Lock-Verbinder 300 verbunden, wobei die radial vorstehenden Verriegelungselemente 130 hinter die Gewindegänge des Gewindes 322 eingreifen. Damit sind die beiden Verbinder 100, 300 fest miteinander verbunden, wie in den Figuren 4A bis 4D dargestellt.

Der weibliche Luer-Lock-Verbinder 100 kann mit dem männlichen Luer-Lock-Verbinder 300 verbunden werden, da die weiblichen Codierungseinschnitte 140 zu den männlichen Codierungsanschlägen 340 bezüglich ihrer axialen Silhouette passen und insbesondere zueinander invers ausgestaltet sind. Passen die weiblichen Codierungseinschnitte 140 nicht zu den männlichen Codierungsanschlägen 340 steht der weibliche Luer-Lock-Verbinder 100 an den zwei Codierungsanschlägen 340 des männlichen Luer-Lock-Verbinders 300 an und kann nicht mit ihm verbunden werden.

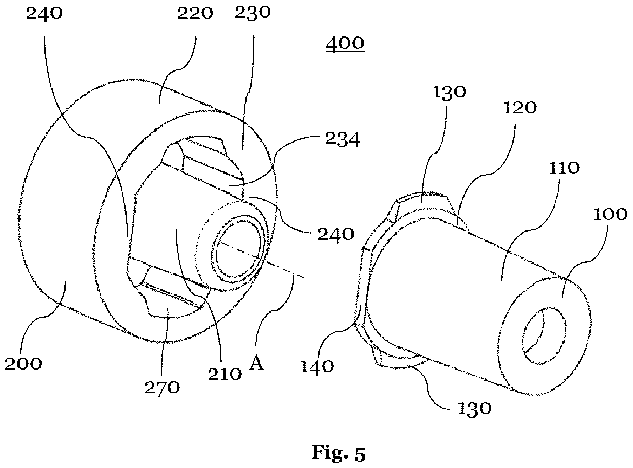

Die Figuren 5 bis 6C zeigen ein weiteres Ausführungsbeispiel eines Luer-Verbindungssystems 400 aus einem männlichen Luer-Verbinder 200 und einem weiblichen Luer-Verbinder 100. In diesem Ausführungsbeispiel sind ein hülsenförmiges Verbindungssperrelement 230 und der Rohrkörper 210 des männlichen Luer-Verbinders 200 einstückig ausgebildet. Dies hat den Vorteil einer einfachen Herstellung und einer besonders robusten Konstruktion. Im Unterschied zu Fig. 2A ist in dieser Ausführungsform der konische Rohrkörper 210 des männlichen Luer-Verbinders 200 radial vom hülsenförmigen Verbindungssperrelement 230 umschlossen.

Die Verbindung wird entsprechend durch axiales Schieben des Rohrkörpers 210 des männlichen Luer-Verbinders 200 in den Aufnahmekörper 110 des weiblichen Luer-Verbinders 100 hergestellt.

Dieses Ausführungsbeispiel kann auch als ein Luer-Lock-Verbindungssystem 400 ausgestaltet sein, bei dem der männliche Luer-Verbinder ein männlicher Luer-Lock-Verbinder 300, der wie oben für den männlichen Luer-Verbinder 200 beschrieben, entsprechend einstückig mit dem Verbindungssperrelement 230 ausgebildet ist.

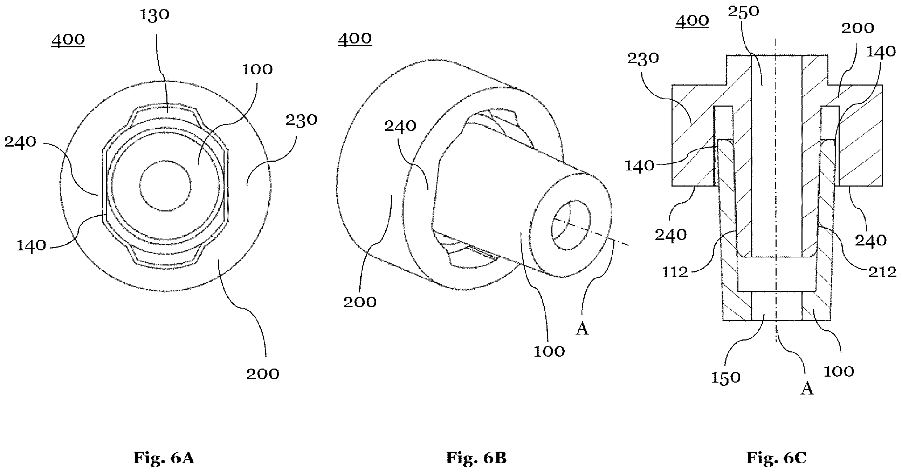

Die Figuren 6A bis 6C zeigen weitere Ansichten des Ausführungsbeispiels von Fig. 5 im verbundenen Zustand. Dabei ist Fig. 6A eine Draufsicht, Fig. 6B eine dreidimensionale Ansicht und Fig. 6C eine Querschnittsseitenansicht des Ausführungsbeispiels von Fig. 5 im verbundenen Zustand. In Fig. 6A sind insbesondere die sich invers entsprechenden Formen bzw. Silhouetten der weiblichen Codierungseinschnitte 140 bzw. männlichen Codierungsanschläge 240 zu erkennen.

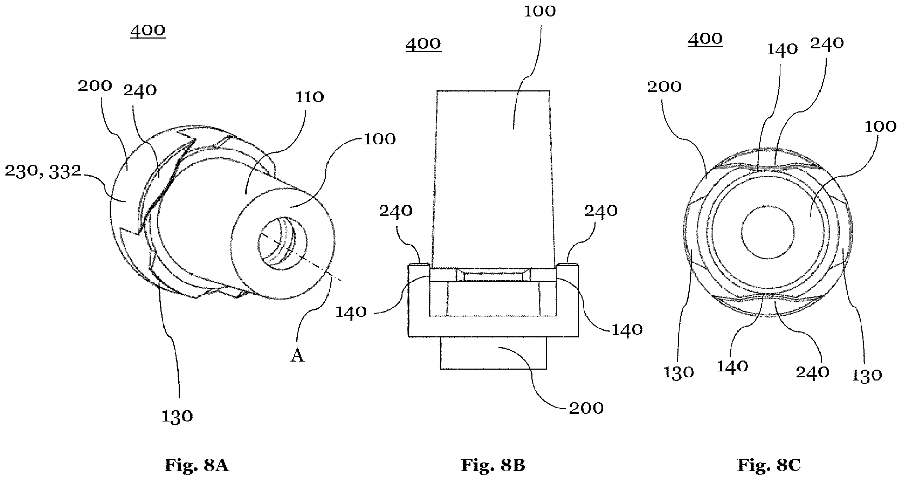

Fig. 7 zeigt ein weiteres Ausführungsbeispiel eines Luer-Verbindungssystems 400. Hierbei sind die männlichen Codierungsanschläge 240 des männlichen Luer-Verbinders 200 und die weiblichen Codierungseinschnitte 140 des weiblichen Luer-Verbinders 100 kreisabschnittsförmig ausgestaltet, wie besonders gut in der Draufsicht nach Fig. 8C zu erkennen ist.

Fig. 8A bis 8C zeigen weitere Ansichten des Ausführungsbeispiels von Fig. 7 im verbundenen Zustand. Dabei ist Fig. 8A eine dreidimensionale Ansicht und Fig. 8B eine Seitenansicht des Ausführungsbeispiels von Fig. 7 im verbundenen Zustand.

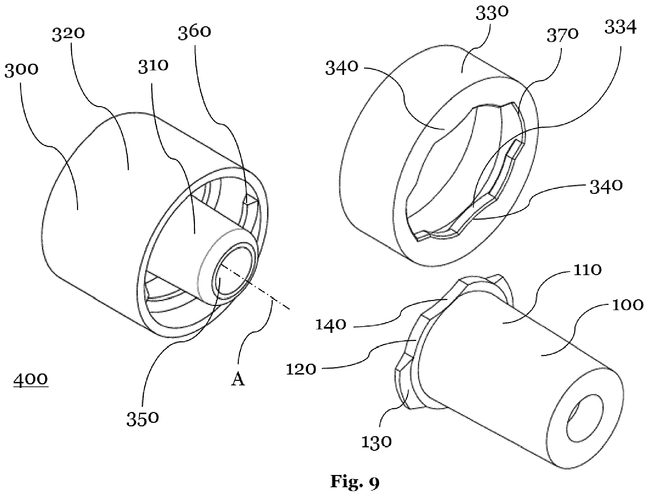

Die Figuren 9 bis 10D zeigen ein weiteres Ausführungsbeispiel eines Luer-Verbindungssystems aus weiblichem Luer-Verbinder 100, ähnlich zu Fig. 7, und einem männlichem Luer-Lock-Verbinder 300, ähnlich zu Fig. 3B, der einen Rohrkörper 310 mit Gewindekörper 320 und ein separates kappenförmiges Verbindungssperrelement 330 umfasst. Der Rohrkörper 310 weist eine Durchgangsöffnung 350 zum Durchleiten von Gasen und Flüssigkeiten auf. Die zwei Codierungsanschläge 340 des Verbindungssperrelements 330 sind hier in ihrer axialen Silhouette kreisabschnittsförmig ausgestaltet, so dass sie mit invers ausgestalteten kreisabschnittsförmigen Codierungseinschnitten 140 des weiblichen Luer-Verbinders 100 zusammenpassen.

Alternativ können der Rohrkörper 310 und das Verbindungssperrelement 330 ähnlich dem Ausführungsbeispiel aus Fig. 5 einstückig ausgebildet sein.

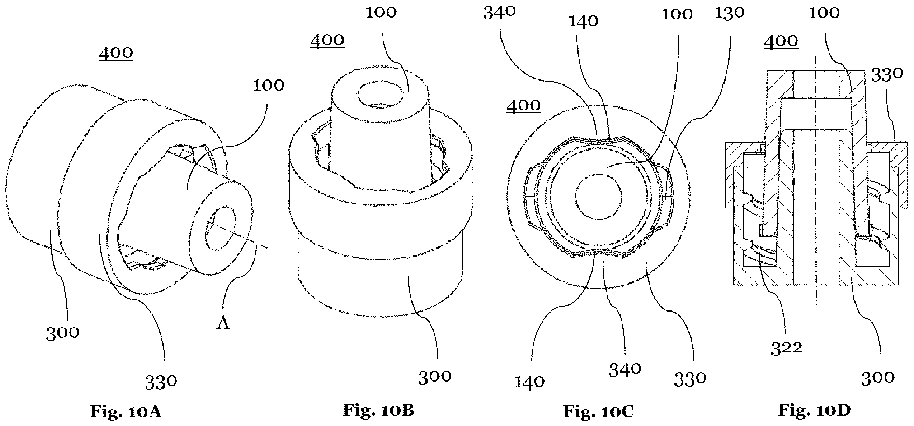

Fig. 10A bis 10D zeigen Ansichten des Ausführungsbeispiels von Fig. 9 im verbundenen Zustand. Dabei sind die Figuren 10A und 10B dreidimensionale Ansichten, Fig. 10C eine Draufsicht, und Fig. 10D eine Querschnittsseitenansicht des Ausführungsbeispiels von Fig. 9 im verbundenen Zustand. In Fig. 10C sind insbesondere die sich invers entsprechenden Formen der weiblichen Codierungseinschnitte 140 bzw. männlichen Codierungsanschläge 340 mit kreisabschnittsförmiger axialer Silhouette gut zu erkennen.

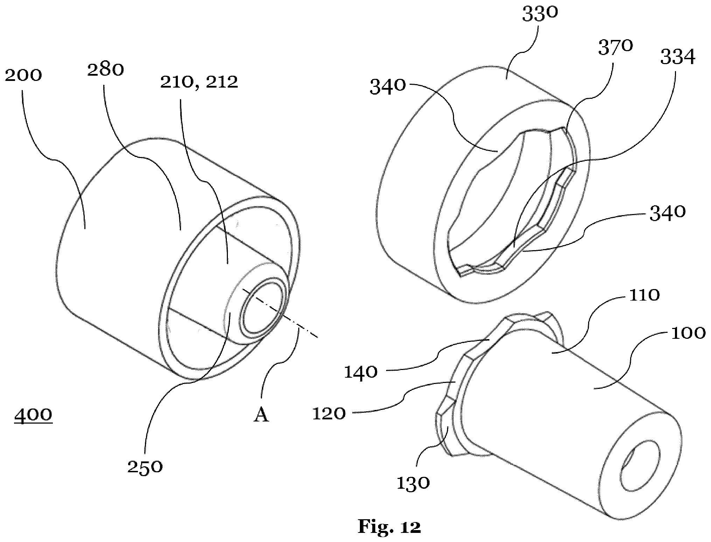

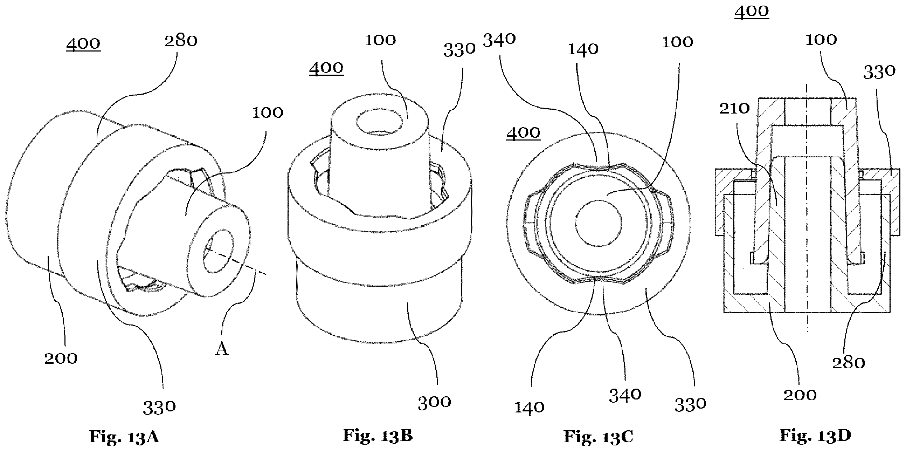

Die Figuren 12 bis 13D zeigen ein weiteres Ausführungsbeispiel eines Luer-Verbindungssystems 400 aus weiblichem Luer-Verbinder 100, ähnlich zu Fig. 7 und Fig. 9, und einem männlichem Luer-Verbinder 200, ähnlich zu dem männlichen Luer-Lock-Verbinder aus Fig. 3B, nur ohne Innengewinde. Der dargestellte Luer-Verbinder 200 ist daher ein Luer-Slip-Verbinder 200. Dieser weist um den eigentlichen Rohrkörper 210 mit konischem Außenmantel 212 herum eine Hülse 280 auf, auf die ein separates kappenförmiges Verbindungssperrelement 330 aufgesteckt werden kann. Dabei kann das Verbindungssperrelement 330, wie dargestellt, bevorzugt dasselbe sein, wie es für das Luer-Lock-Verbindungssystem der Figuren 9 - 10D verwendet wird. Das Verbindungssperrelement 330 ist somit universell verwendbar, was u.a. die Herstellkosten senkt. Der Rohrkörper 210 weist, wie gehabt, eine Durchgangsöffnung 250 zum Durchleiten von Gasen und Flüssigkeiten auf. Die Hülse 280 kann, wie in Fig. 13D ersichtlich, bevorzugt einstückig mit dem Rohrkörper 210 des männlichen Luer-Verbinders 200 ausgeführt sein. Die zwei Codierungsanschläge 340 des Verbindungssperrelements 330 sind hier in ihrer axialen Silhouette kreisabschnittsförmig ausgestaltet, so dass sie mit invers ausgestalteten kreisabschnittsförmigen Codierungseinschnitten 140 des weiblichen Luer-Verbinders 100 zusammenpassen.

Alternativ können der Rohrkörper 210 und das Verbindungssperrelement 330 ähnlich dem Ausführungsbeispiel aus Fig. 5 einstückig ausgebildet sein, was jedoch fertigungstechnisch schwieriger zu realisieren ist, als die dargestellte eine mehrteilige Lösung mit separatem Verbindungssperrelement 330.

Die Fig. 13A bis 13D zeigen Ansichten des Ausführungsbeispiels von Fig. 12 im verbundenen Zustand. Dabei sind die Figuren 13A und 13B dreidimensionale Ansichten, Fig. 13C eine Draufsicht, und Fig. 13D eine Querschnittsseitenansicht des Ausführungsbeispiels von Fig. 12 im verbundenen Zustand. In Fig. 13C sind insbesondere die sich invers entsprechenden Formen der weiblichen Codierungseinschnitte 140 bzw. männlichen Codierungsanschläge 340 mit kreisabschnittsförmiger axialer Silhouette gut zu erkennen.

Bei sämtlichen Ausführungsbeispielen, die ein Luer-Verbindungssystem 400 betreffen, das einen weiblichen Luer-Verbinder 100 mit Verriegelungselementen 130 aufweist, kann der männliche Luer-Verbinder 200, 300 entsprechend, wie beispielsweise in Fig. 5 dargestellt, eine der Form des Verriegelungselements 130 aufweisen, die eine inverse Aussparung 270, 370 für die Verriegelungselemente 130 umfasst.

Durch die verschiedenen Ausführungsbeispiele der vorliegenden Erfindung wird ein Luer-Verbindungssystem 400 exemplarisch erläutert, durch das Verwechslungen zwischen Luer-Verbindern 100, 200, 300 wirksam verhindert werden können und damit die Patientensicherheit einfach und kostengünstig erheblich gesteigert werden kann.

## Patentansprüche

1. Männlicher Luer-Lock-Verbinder (300) aufweisend:
a. einen Rohrkörper (310) mit einem konischen Außenmantel; und
b. ein Verbindungssperrelement (330);
c. wobei das Verbindungssperrelement (330) einen Raum um den Rohrkörper (310) herum begrenzt;
d. in axialer Richtung (A) einen Codierungsanschlag (340) bildet; und
e. dazu eingerichtet ist, eine Verbindung des männlichen Luer-Lock-Verbinders (300) mit einem weiblichen Standard-Luer-Verbinder und einem weiblichen Standard-Luer-Lock-Verbinder mechanisch zu verhindern, wobei weibliche Standard-Luer-Verbinder einer Kanüle mit ihrem Bund an dem Codierungsanschlag (340) anstehen; und
f. und der männliche Luer -Lock-Verbinder (300) weiterhin einen Gewindekörper (320) mit einem Innengewinde (362) aufweist, wobei der Codierungsanschlag (340) des Verbindungssperrelements (330) am äußeren Ende des Gewindekörpers (330) angebracht ist.

2. Männlicher Luer-Lock-Verbinder (300) nach Anspruch 1, wobei das Verbindungssperrelement (330) zwei gegenüberliegende Codierungsanschläge (340) aufweist.

3. Männlicher Luer-Lock-Verbinder (300) nach Anspruch 2, wobei die zwei gegenüberliegenden Codierungsanschläge (340) durch zwei gegenüberliegende Vorsprünge geformt werden.

4. Männlicher Luer-Lock-Verbinder (300) nach einem der Ansprüche 2 oder 3, wobei die Codierungsanschläge (340) in ihrer Form gleich ausgestaltet sind.

5. Männlicher Luer-Lock-Verbinder (300) nach einem der Ansprüche 2 oder 3, wobei die Codierungsanschläge (340) in ihrer Form unterschiedlich ausgestaltet sind.

6. Männlicher Luer-Lock-Verbinder (300) nach einem der Ansprüche 1 bis 5, wobei der Codierungsanschlag (340), in axialer Richtung (A) gesehen, eine gerade, dreieckige, rechteckige, kreisabschnittsförmige oder trapezförmige Silhouette aufweist.

7. Männlicher Luer-Lock-Verbinder (300) nach einem der Ansprüche 1 bis 6, wobei das Verbindungssperrelement (330) separat vom Rohrköper (310) hergestellt ist und eine hohlzylindrische Aufnahme (334) aufweist, mit der das Verbindungssperrelement (330) auf dem Gewindekörper (320) des männlichen Luer-Lock-Verbinders (300) verbindbar ist.

8. Männlicher Luer-Lock-Verbinder (300) nach einem der Ansprüche 1 bis 6, wobei das Verbindungssperrelement (330) einstückig mit dem männlichen Luer-Lock-Verbinder (200 300) ausgebildet ist.

9. Weiblicher Luer-Lock-Verbinder (100), aufweisend
a. einen hohlen Aufnahmekörper (110) mit einer konischen Aufnahme (112), und
b. einen Bund (120) am äußeren Ende des Aufnahmekörpers (110); wobei
c. der Bund (120) mindestens einen in den Bund (120) radial nach innen eingeschnittenen Codierungseinschnitt (140) aufweist und
d. weiterhin ein oder mehrere vom Bund (120) radial nach außen vorstehende Verriegelungselemente (130) aufweist.

10. Weiblicher Luer-Lock-Verbinder (100) nach Anspruch 9, wobei der Codierungseinschnitt (140), in axialer Richtung (A) gesehen, eine gerade, dreieckige, rechteckige, kreisabschnittsförmige oder trapezförmige Silhouette aufweist.

11. Weiblicher Luer-Lock-Verbinder (100) nach einem der Ansprüche 9 oder 10, wobei der Bund (120) entweder
a. zwei radial gegenüberliegend angeordnete Codierungseinschnitte (140); oder
b. zwei asymmetrisch angeordnete Codierungseinschnitte (140) aufweist.

12. Verbindungssystem (400) aufweisend einen männlichen Luer-Lock-Verbinder (300) nach einem der Ansprüche 1 bis 8 und einen weiblichen Luer-Lock-Verbinder (100) nach einem der Ansprüche 9 bis 11, wobei der eine Codierungseinschnitt (140) oder die mehreren Codierungseinschnitte (140) des weiblichen Luer-Lock-Verbinders (100) invers zum einen Codierungsanschlag (240,340) oder den mehreren Codierungsanschlägen (240,340) des männlichen Luer-Lock-Verbinders (200,300) ausgestaltet ist oder sind.

## Claims

1. A male luer lock connector (300) comprising:
a. a tube body (310) having a conical outer sleeve; and
b. a connection blocking member (330);
c. wherein the connection blocking member (330) delimits a space around the tube body (310);
d. forms a coding stop (340) in axial direction (A); and
e. is configured to mechanically prevent connection of the male luer lock connector (300) to a standard female luer connector and a standard female luer lock connector, wherein standard female luer connectors of a cannula abut the coding stop (340) with their collar; and
f. and the male luer lock connector (300) further comprises a threaded body (320) having an internal thread (362), wherein the coding stop (340) of the connection blocking member (330) is attached to the outer end of the threaded body (330).

2. The male luer lock connector (300) of claim 1, wherein the connection blocking member (330) comprises two opposing coding stops (340).

3. The male luer lock connector (300) of claim 2, wherein the two opposing coding stops (340) are formed by two opposing protrusions.

4. The male luer lock connector (300) of any one of claims 2 or 3, wherein the coding stops (340) are configured to have the same shape.

5. The male luer lock connector (300) of any one of claims 2 or 3, wherein the coding stops (340) are configured to have a different shape.

6. The male luer lock connector (300) of any one of claims 1 to 5, wherein the coding stop (340) has a straight, triangular, rectangular, circular-section-shaped, or trapezoidal silhouette when viewed in axial direction (A).

7. The male luer lock connector (300) of any one of claims 1 to 6,
wherein the connection blocking member (330) is manufactured separately from the tube body (310) and comprises a hollow cylindrical receptacle (334) with which the connection blocking member (330) is connectable to the threaded body (320) of the male luer lock connector (300).

8. The male luer lock connector (300) of any one of claims 1 to 6, wherein the connection blocking member (330) is integrally formed with the male luer lock connector (200, 300).

9. A female luer lock connector (100) comprising
a. a hollow receiving body (110) having a conical receptacle (112), and
b. a collar (120) at the outer end of the receiving body (110); wherein
c. the collar (120) comprises at least one codification notch (140) cut radially inwardly into the collar (120), and
d. further comprises one or more locking elements (130) protruding radially outwardly from the collar (120).

10. The female luer lock connector (100) of claim 9, wherein the codification notch (140) has a straight, triangular, rectangular, circular-section-shaped, or trapezoidal silhouette when viewed in axial direction (A).

11. The female luer lock connector (100) of any one of claims 9 or 10, wherein the collar (120) comprises either
a. two codification notches (140) arranged radially opposite; or
b. two codification notches (140) arranged asymmetrically.

12. A connection system (400) comprising a male luer lock connector (300) according to any one of claims 1 to 8 and a female luer lock connector (100) according to any one of claims 9 to 11, wherein the one or more codification notches (140) of the female luer lock connector (100) are configured inversely to the one or more coding stops (240,340) of the male luer lock connector (200 300).

## Revendications

1. Connecteur Luer-Lock mâle (300) comprenant :
a. un corps tubulaire (310) avec une enveloppe extérieure conique ; et
b. un élément de blocage de connexion (330) ;
c. dans lequel l'élément de blocage de connexion (330) limite un espace autour du corps tubulaire (310) ;
d. forme dans la direction axiale (A) une butée de codage (340) ; et
e. est agencé pour empêcher mécaniquement une connexion du connecteur Luer-Lock mâle (300) avec un connecteur Luer standard femelle et avec un connecteur Luer-Lock standard femelle, où des connecteurs Luer standard femelles d'une canule butent avec leur collerette contre la butée de codage (340) ;
f. et le connecteur Luer-Lock mâle (300) comporte en outre un corps fileté (320) avec un filetage intérieur (362), la butée de codage (340) de l'élément de blocage de connexion (330) étant disposée à l'extrémité extérieure du corps fileté (330).

2. Connecteur Luer-Lock mâle (300) selon la revendication 1, dans lequel l'élément de blocage de connexion (330) comporte deux butées de codage en vis-à-vis (340).

3. Connecteur Luer-Lock mâle (300) selon la revendication 2, dans lequel les deux butées de codage en vis-à-vis (340) sont formées par des saillies en vis-à-vis.

4. Connecteur Luer-Lock mâle (300) selon une des revendications 2 ou 3, dans lequel les butées de codage (340) sont conçues de manière identique dans leur forme.

5. Connecteur Luer-Lock mâle (300) selon une des revendications 2 ou 3, dans lequel les butées de codage (340) sont conçues de manière différente dans leur forme.

6. Connecteur Luer-Lock mâle (300) selon une des revendications 1 à 5, dans lequel la butée de codage (340), vue dans la direction axiale (A), présente une silhouette rectiligne, triangulaire, rectangulaire, en forme d'arc de cercle ou trapézoïdale.

7. Connecteur Luer-Lock mâle (300) selon une des revendications 1 à 6, dans lequel l'élément de blocage de connexion (330) est fabriqué séparément du corps tubulaire (310) et comporte un logement cylindrique creux (334) avec lequel l'élément de blocage de connexion (330) peut être connecté sur le corps fileté (320) du connecteur Luer-Lock mâle (300).

8. Connecteur Luer-Lock mâle (300) selon une des revendications 1 à 6, dans lequel l'élément de blocage de connexion (330) est conçu d'un seul tenant avec le connecteur Luer-Lock mâle (200 300).

9. Connecteur Luer-Lock femelle (100), comprenant
a. un corps récepteur creux (110) avec un logement conique (112), et
b. une collerette (120) à l'extrémité extérieure du corps récepteur (110) ; dans lequel
c. la collerette (120) comporte au moins une encoche de codage (140) pratiquée dans la collerette (120) radialement vers l'intérieur et
d. un ou plusieurs éléments de verrouillage (130) font en outre saillie radialement vers l'extérieur par rapport à la collerette (120).

10. Connecteur Luer-Lock femelle (100) selon la revendication 9, dans lequel l'encoche de codage (140), vue dans le sens axial (A), présente une silhouette rectiligne, triangulaire, en forme d'arc de cercle ou trapézoïdale.

11. Connecteur Luer-Lock femelle (100) selon une des revendications 9 ou 10, dans lequel la collerette (120) comporte soit
a. deux encoches de codage (140) disposées radialement en vis-à-vis ; soit
b. deux encoches de codage (140) disposées de manière asymétrique.

12. Système de connexion (400) comportant un connecteur Luer-Lock mâle (300) selon une des revendications 1 à 8 et un connecteur Luer-Lock femelle (100) selon une des revendications 9 à 11, dans lequel l'une encoche de codage (140) ou les plusieurs encoches de codage (140) du connecteur Luer-Lock femelle (100) est conçue ou sont conçues à l'inverse d'une butée de codage (240,340) ou des plusieurs butées de codage (240,340) du connecteur Luer-Lock mâle (200 300).
